# EUROPÄISCHE PATENTSCHRIFT

(11) **EP 2 510 234 B1**
(45) Veröffentlichungstag und Bekanntmachung des Hinweises auf die Patenterteilung: **06.03.2019**
(21) Anmeldenummer: 10805582.3
(22) Anmeldetag: 10.12.2010
(51) Int. Cl.: F04B 1/02, F04B 3/00, F04B 7/00, F04B 25/00, A61M 5/142, A61M 5/36

(54) **KONTINUIERLICH FÖRDERNDE INFUSIONSPUMPE**
CONTINUOUSLY CONVEYING INFUSION PUMP
POMPE À PERFUSION À TRANSPORT CONTINU

(30) Priorität: 11.12.2009 DE 102009057792
(43) Veröffentlichungstag der Anmeldung: 17.10.2012
(73) Patentinhaber: LENUS Infusionstechnik GmbH & Co.KG, 24118 Kiel (DE)
(72) Erfinder: Kölln, Harm, 24159 Kiel (DE)
(74) Vertreter: Lobemeier, Martin Landolf
(86) Internationale Anmeldenummer: PCT/DE2010/001438
(87) Internationale Veröffentlichungsnummer: WO 2011/069494

(56) Entgegenhaltungen:
- WO-A1-2008/141337
- US-A1- 2007 261 553

## Beschreibung

Die Erfindung betrifft eine Infusionspumpe zum kontinuierlichen Fördern eines Fluids, mit einem Einlass, einem Auslass, einen in einer ersten Kammer verschieblich gelagerten ersten Kolben, und einem in einer zweiten Kammer verschieblich gelagerten zweiten Kolben. Insbesondere betrifft die Erfindung eine Infusionspumpe mit der Kleinstmengen eines Medikaments genau und mit einer beständig gleichbleibenden Fluss- bzw. Förderrate appliziert werden können.

Infusionspumpen sind beispielsweise aus der WO 2008/141337 A1 bekannt, die eine kontinuierlich fördernde Infusionspumpe offenbart, bei der ein Fluid mittels Kolben wechselseitig aus zwei Kammern getrieben wird, wobei beim Umschalten der Bewegungsrichtung der Kolben kurzzeitig ein Fördern des Fluids aus beiden Kammern gleichzeitig erfolgt. Eine weitere Infusionspumpe ist aus der US 2007/0261553 A1 bekannt.

Aus der EP 0 458 114 B1 ist bereits eine Infusionspumpe bekannt, bei der zwei mit jeweils einem Kolben ausgestattete Zylinder mithilfe von vier Ventilen abwechselnd aus einem Reservoir mit einem Medikament befüllt und das Medikament an den Patienten abgebend abwechselnd entleert werden. Durch das Umschalten der Medikamentenversorgung von einem entleerten auf einen gefüllten Zylinder kann mit relativ einfachen Mitteln eine annähernd kontinuierliche Medikamentenförderung erreicht werden.

Nachteil dieser Infusionspumpe ist jedoch, dass aufgrund des Ventilspiels zusätzliches Volumen im Ventil erzeugt wird, wenn das Ventil geöffnet wird, wodurch eine Minderförderung des Medikaments erfolgt. Andererseits wird dieses Mindervolumen beim Schließen des Ventils wieder verdrängt, sodass eine Mehrförderung die Folge ist.

Über einen längeren Zeitraum betrachtet entspricht die Medikamentenabgabe zwar dem vom Arzt vorgesehenen Therapieplan. Allerdings kann unter Umständen bereits eine zeitlich nur kurze Minder- oder Mehrversorgung des Patienten mit dem von der Infusionspumpe geförderten Medikament - abhängig vom Medikament - dazu führen, dass der gewünschte Therapieerfolg nicht erreicht wird.

Andere im medizinischen Umfeld verwendete Pumpeinrichtungen sind beispielsweise aus der DE 30 32 475 A1 oder aus der US 4 808 077 A bekannt. Diese eignen sich jedoch nicht als gattungsgemäße Infusionspumpen, da diese die Anforderungen der E DIN 60601-2-24 im Hinblick auf eine Rückwärtsförderung und einen Schutz vor Überdosierung bei unkontrolliertem Fluss nicht erfüllen.

Aufgabe der Erfindung ist es daher, eine Infusionspumpe zu schaffen, mit der auch sehr kleine Flüssigkeitsmengen, bevorzugt im Mikroliterbereich, sehr genau und kontinuierlich zu fördern.

Diese Aufgabe wird durch die Infusionspumpe mit den Merkmalen von Anspruch 1 und die Infusionspumpe mit den Merkmalen von Anspruch 2 gelöst. Die Unteransprüche geben vorteilhafte Ausgestaltungen der Erfindung wieder.

Die kontinuierlich fördernde Infusionspumpe nach der Erfindung stellt durch die Anordnung der ersten, als Förderkammer bezeichneten Kammer und der zweiten, als Ausgleichskammer bezeichneten Kammer zusammen mit volumenneutralen Ventilen sicher, dass die Medikamentengabe nur von der Ansteuerung der Aktoren der vorgenannten Elemente abhängig und damit bei identischen Ansteuerungszyklen bei jeder gewünschten Förderrate kontinuierlich ist.

Die Erfindung wird anhand eines besonders bevorzugt ausgestalteten Ausführungsbeispiels näher erläutert. Es zeigen:
- Fig. 1: eine schematische Schnittansicht durch ein besonders bevorzugtes Ausführungsbeispiel der erfindungsgemäßen Infusionspumpe;
- Fig. 2: eine schematische Schnittansicht der Infusionspumpe aus Fig. 1 in einem ersten Schaltzustand; und
- Fig. 3: eine schematische Schnittansicht der Infusionspumpe aus Fig. 1 in einem zweiten Schaltzustand.

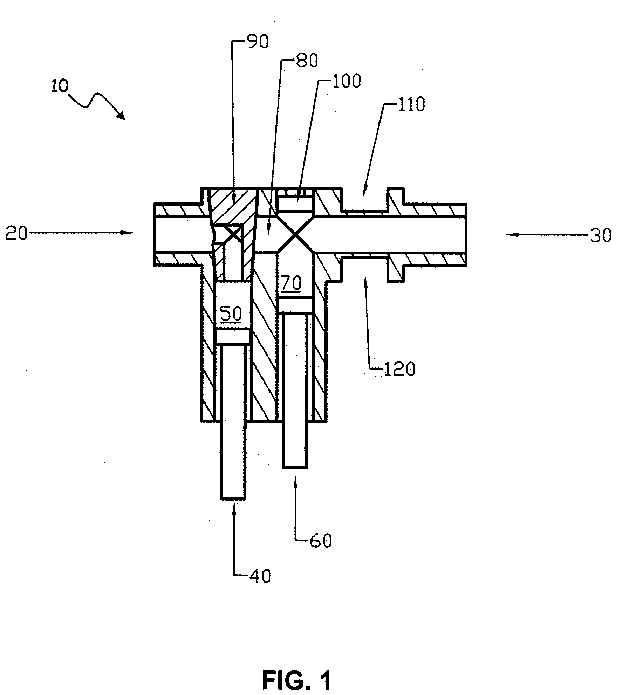

Fig. 1 zeigt eine schematische Schnittansicht durch ein besonders bevorzugtes Ausführungsbeispiel der Infusionspumpe nach der Erfindung. Die Infusionspumpe 10 weist einen Einlass 20 zum Einleiten von Fluid, z.B. einem Medikament, aus einem (nicht dargestellten) Reservoir in die Infusionspumpe 10, sowie einen das Fluid aus der Infusionspumpe 10 an den Patienten abgebenden Auslass 30 auf. Ein- und Auslass 20, 30 der Infusionspumpe sind bevorzugt fluchtend, an sich gegenüberliegenden Seiten der Infusionspumpe 10 angeordnet.

Die Infusionspumpe 10 weist eine erste Kammer 50 mit einem ersten Kolben 40 und eine in Flussrichtung nachfolgende, bevorzugt parallel benachbarte zweite Kammer 70 mit einem zweiten Kolben 60 auf, wobei die erste Kammer 50 und der erste Kolben 40 auch Förderkammer 50 bzw. Förderkolben 60 und die zweite Kammer 70 und der zweite Kolben 60 auch Ausgleichskammer 70 und Ausgleichskolben 60 genannt werden. Die erste Kammer 50 ist mit dem Einlass 20 (oder einem Ansaugkanal) verbunden, wohingegen die zweite Kammer 70 mit dem Auslass 30 (oder einem Ausstoßkanal) verbunden ist. Die Kammern 50, 70 sind untereinander durch einen Verbindungskanal 80 verbunden, wobei der Verbindungskanal 80 bevorzugt mit dem Auslass 30 fluchtend angeordnet ist. Besonders bevorzugt sind Einlass 20, Verbindungskanal 80 und Auslass 30 fluchtend angeordnet.

Die Kolben 40, 60 sind in der ersten, als Förderkammer bezeichneten Kammer 50 bzw. der zweiten, als Ausgleichskammer bezeichneten Kammer 70 verschieblich gelagert, wobei der Zwischenraum zwischen den Kolben 40, 60 und der Innenwandung der Kammern 50, 70 gegebenenfalls mit geeigneten Mitteln abgedichtet ist. Die durch die Kolben 40, 60 in den Kammern 50, 70 gebildeten Kolbenpumpen werden mittels (nicht dargestellter) geeigneter Aktoren betätigt.

Erfindungsgemäß ist ein bevorzugt als 2-Weg-Hahn ausgebildetes Stellelement 90 vorgesehen, wobei vorzugsweise die Hülse des Hahns 90 durch das Gehäuse der Infusionspumpe 10 ausgebildet ist. Das Küken des Hahns 90 ist in der Hülse abgedichtet gelagert. Das Küken kann auch als Teil eines 3-Wege-Hahn ausgebildet sein, damit zur Funktionsausübung des Stellelements 90 nur eine geringfügige Drehung des 3-Wege-Hahns um wenige Winkelgrade notwendig ist.

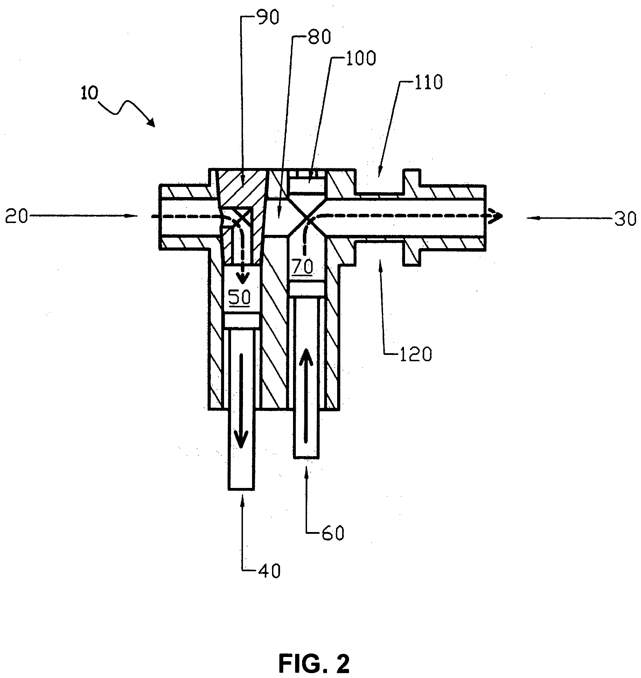

Das Stellelement 90 ist nämlich so ausgebildet, dass in einer ersten Position des Stellelements 90 der Einlass 20 mit der ersten Kammer 50 kommunizierend verbunden ist, wohingegen die Kommunikation zwischen der ersten Kammer 50 und der zweiten Kammer 70 durch das Stellelement 90 blockiert ist. In dieser Position kann die erste Kammer 50 aus dem Reservoir durch eine Abwärtsbewegung des ersten Kolbens 40 befüllt werden und die zweite Kammer 70 ihren Inhalt durch eine Aufwärtsbewegung des zweiten Kolbens 60 an den Patienten durch den Auslass 30 als kontinuierliche Förderung abgeben (siehe Fig. 2).

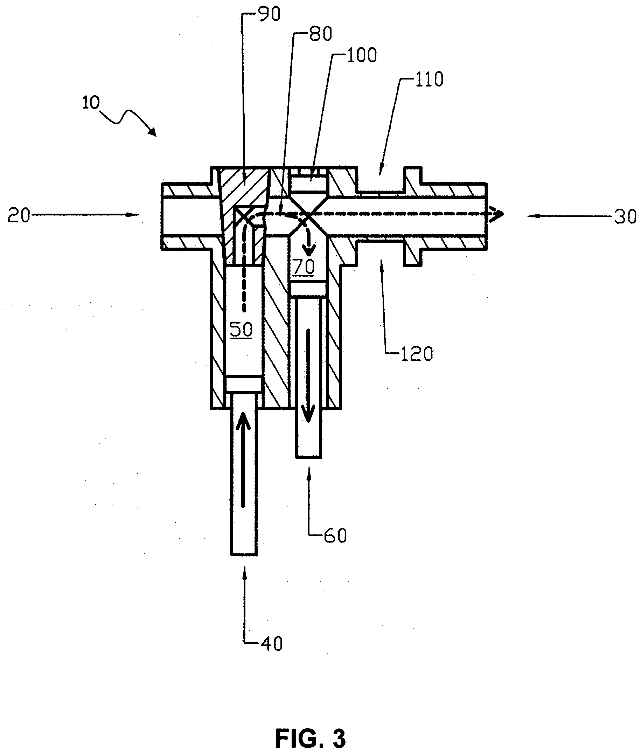

In einer zweiten, beispielsweise um 180° gedreheten Position des 2-Wegehahns 90 (bzw. dessen Kükens 90) ist die Kommunikation zwischen dem Einlass 20 und der ersten Kammer 50 durch das Küken 90 blockiert, wobei jedoch eine Kommunikation zwischen der ersten Kammer 50 und der zweiten Kammer 70 über den Verbindungskanal 80 hergestellt wird. Durch Aufwärtsbewegung des ersten Kolbens 40 wird das aus der ersten Kammer 50 verdrängte Fluid in die zweite Kammer 70 geleitet, die bei gleichzeitiger Abwärtsbewegung des zweiten Kolbens 60 gefüllt wird (siehe Fig. 3).

Alternativ kann das Stellelement aber auch anstelle des Hahns 90 durch zwei (nicht dargestellte) Ventile gebildet sein, wobei ein erstes Ventil zwischen dem Einlass und der ersten Kammer und ein zweites Ventil zwischen der ersten Kammer und der zweiten Kammer angeordnet sind. Die Ventile werden dabei wechselseitig geschaltet, sodass die Funktion des oben dargestellten Hahns von den zwei Ventilen übernommen wird.

Als weitere bevorzugte Ausgestaltung der Alternative ist es denkbar, dass das erste Ventil als Rückschlagventil und das zweite Ventil als durch einen Aktuator betätigtes Ventil ausgebildet ist. Wiederum als besonders bevorzugte Alternative zu dieser Ausgestaltung kann das erste Ventil als Rückschlagventil und das zweite Ventil als Siphon ausgestaltet sein, dass bevorzugt erst bei einem Differenzdruck von > 100 hPa öffnet.

Dabei sind die Bewegungen der beiden Kolben 40, 60 durch eine (nicht dargestellte) Steuerung derart koordiniert, dass das durch Entleeren der ersten Kammer 50 und Befüllen der zweiten Kammer 70 entstehende, ausgetriebene Differenzvolumen, demjenigen Volumen entspricht, das für eine kontinuierlich Förderung der gewünschten Medikamentengabe erforderlich ist. Durch die Kontinuität der Förderung ist sichergestellt, dass das Medikament immer mit Druck in das Gewebe infundiert wird, wie es z.B. bei der "Convection Enhanced Delivery" (CED) Methode gefordert ist.

Im Ausführungsbeispiel ist die verbundene Einheit zum Anschluss zur Druckmessung als Kolben 100 ausgeführt. Der Kolben 100 sitzt auf einem nicht dargestellten, geeigneten Kraftsensor, z.B. einen Biegebalken. Aus der Kraft, mit der dieser Kolben 100 in Position gehalten wird, läst unter Berücksichtigung der Kolbenfläche der Druck in der Ausgleichskammer 70, die mit dem Ausflusskanal 30 verbunden ist, ermitteln. Alternativ kann anstelle des vorgenannten verschieblich gelagerten dritten Kolbens 100 auch eine inflatierbare/deflatierbare Membran zur Druckmessung verwendet werden. Die Druckmessung ist notwendig, um z.B. eine Okklusion zu detektieren, wobei bei einem Druckanstieg über einen vorbestimmten Wert vorgesehen ist, dass ein Alarm ausgegeben wird.

Schließlich ist auch ein Bereich 110, 120 zum Anschluss eines Detektors zur Blasenerkennung im Fluid an die Infusionspumpe vorgesehen. Die Blasenerkennung kann beispielsweise mittels Ultraschallmessung mit einer Frequenz im MHz Bereich vorgesehen sein oder durch optische Messung unter Ausnutzung der unterschiedlichen Brechungsindizes von Flüssigkeit und Gas vorgenommen werden. Dafür ist eine Aufnahmekammer 110 für den Schall- oder Licht-Sender und eine Aufnahmekammer 120 für dem Schall- oder Licht-Empfänger in den Pumpenträger der Infusionspumpe 10 integriert. Der Auslass 30 hat im Ausführungsbeispiel einen rechteckigen Querschnitt um ungewünschte Reflexion, oder Brechung an nicht ebenen Grenzflächen von Licht oder Schall zu vermeiden.

Zusammenfassend wird noch einmal der Ablauf der Verfahrensablauf der von der Steuerung gesteuerten Infusionspumpe beschrieben:
Das Küken 90 verschließt den Verbindungskanal 80 und hat die Förderkammer 50 über den Einlass 20 mit dem (nicht dargestellten) Flüssigkeitsvorrat verbunden. Der Ausgleichskolben 60 wird in diesem Zustand mit der Geschwindigkeit nach innen (d.h. in der Zeichnungsebene nach oben) gedrückt, die notwendig ist um die gewünschte Flüssigkeitsmenge kontinuierlich durch den Auslass 30 zu fördern. Gleichzeitig wird der Förderkolben 50 mit der maximal vorgesehenen Geschwindigkeit nach außen (d.h. in der Zeichnungsebene nach unten) gezogen. Dabei füllt sich die Förderkammer 50 mit dem Fluid, z.B. ein Medikament, aus dem Flüssigkeitsvorrat. Diese Flüssigkeitsströme sind aufgrund des geschlossenen Verbindungskanals 80 voneinander unabhängig. Nachdem die Vorratskammer 50 gefüllt ist, wird der Förderkolben 40 nicht mehr bewegt. Danach wird das Küken 90 im Ausführungsbeispiel um 180° gedreht, bis der Einlass 20 geschlossen und der Verbindungskanal 90 geöffnet ist, während der Ausgleichskolben 60 weiter mit konstanter Geschwindigkeit nach innen gedrückt wird. Diese Vorgänge stellen sicher, dass die Ausfließgeschwindigkeit des Fluids im Auslass 30 nicht verändert wird.

Darauf wird der Förderkolben 40 derart nach innen (d.h. in der Zeichnungsebene nach oben) gedrückt, dass eine höhere Flüssigkeitsmenge aus der Förderkammer 50 ausgetrieben wird, als zur konstanten Förderung durch den Auslass 30 notwendig ist. Gleichzeitig wird der Ausgleichskolben 60 mit derjenigen Geschwindigkeit nach außen (d.h. in der Zeichnungsebene nach unten) gezogen und damit das Volumen der Ausgleichskammer 70 vergrößert, wodurch Fluid, das aus dem Verbindungskanal 80 austritt, in der Ausgleichskammer 70 aufgenommen wird. Das geschieht in dem Maß, das notwendig ist, um die Ausfließgeschwindigkeit der Flüssigkeit am Auslass 30 weiter konstant zu halten.

Nachdem die Förderkammer 70 entleert ist, wird der Förderkolben 40 nicht mehr bewegt und der Ausgleichskolben 60 wird wieder mit der Geschwindigkeit nach innen (d.h. in der Zeichnungsebene nach oben) gedrückt, die erforderlich ist, um weiter die konstante Ausfließgeschwindigkeit des Fluids im Auslass 30 aufrecht zu halten. Dabei wird das beim Entleeren der Förderkammer 40 in der Ausgleichskammer 70 gesammelte Fluid ausgetrieben.

Jetzt wird das Küken 90 wieder um 180° gedreht, bis der Verbindungskanal 80 verschlossen ist und die Förderkammer 50 mit dem Einlass 20 verbunden ist. Danach wird der Förderkolben 40 wieder mit der maximal vorgesehenen Geschwindigkeit nach außen (d.h. in der Zeichnungsebene nach unten) gezogen und die Förderkammer 50 füllt sich erneut und ein erneuter Steuerzyklus setzt wie oben beschrieben ein. Der kontinuierliche Flüssigkeitsausstoß im Auslass 30 kann derart solange aufrecht erhalten werden, bis der Flüssigkeitsvorrat, an den der Einlass angeschlossen ist, aufgebraucht ist.

Durch die Eigenschaft der erfindungsgemäßen kontinuierlich fördernde Infusionspumpe 10, dass augrund der Konstruktion im Ausführungsbeispiel zu keiner Zeit eine Situation entstehen kann, bei der "freeflow" (ungesteuerter Medikamentenfluss aufgrund von Druckdifferenz zwischen dem Flüssigkeitsvorrat und dem Patientenzugang) möglich ist, kann auf zusätzliche Maßnahmen bei Druckdifferenz durch Schwerkraft, Temperaturschwankung oder anderen Einflüssen verzichtet werden.

Jederzeit ist es möglich, die Ausfließgeschwindigkeit der Flüssigkeit zwischen der minimalen und maximalen vorgesehenen Grenze durch entsprechende Ansteuerung der Aktoren zu ändern. Diese Grenzen sind von der Ausführung der Pumpe 10, wie z.B. Leistung der gewählten Aktoren und Maße der Pumpe, welche die Größe der Kammern 50, 70 und die Durchmesser der Kolben 40, 60 bestimmen, abhängig.

Schließlich ermöglicht die Erfindung auch eine Ausgestaltung der Infusionspumpe 10 als Einmalartikel in steriler Ausführung. Die einfache Konstruktion der erfindungsgemäßen Infusionspumpe 10 ermöglicht nämlich die Herstellung in Form eines Spritzgussteils, wodurch eine kostengünstige Produktion ermöglicht ist und die Infusionspumpe 10 als Einmalartikel Verwendung finden kann.

Die Pumpe erfüllt unter anderem auch die Anforderung zum Einsatz der Arzneimittelgabe direkt in das Hirngewebe nach der "Convection Enhanced Delivery" (CED) Methode, die gegenüber der nicht kontinuierlichen Arzneimittelgabe große Vorteile aufweist.

## Patentansprüche

1. Infusionspumpe (10) zum kontinuierlichen Fördern eines Fluids, mit
- einem Einlass (20),
- einem Auslass (30),
- einen in einer ersten Kammer (50) verschieblich gelagerten ersten Kolben (40), und
- einem in einer zweiten Kammer (70) verschieblich gelagerten zweiten Kolben (60),
- einem die erste Kammer (50) mit der zweiten Kammer (70) verbindenden Verbindungskanal (80), wobei die erste Kammer (50) mit dem Einlass (20) und die zweite Kammer (70) mit dem Auslass (30) verbunden ist,
- einem als 2-Wege- oder 3-Wege-Hahn ausgebildeten Stellelement (90), das in einer ersten Position den Einlass (20) mit der ersten Kammer (50) und in einer zweiten Position die erste Kammer (50) mit der zweiten Kammer (70) kommunizierend verbindet, wobei in der ersten Position die Kommunikation zwischen der ersten Kammer (50) und der zweiten Kammer (70) und in der zweiten Position die Kommunikation zwischen dem Einlass (20) und der ersten Kammer (50) blockiert ist,
- einer auf den ersten Kolben (40), den zweiten Kolben (60) und das Stellelement (90) wirkenden Steuerung, sodass bei Stellung des Stellelements (90) in der ersten Position die erste Kammer (50) befüllt wird, während die zweite Kammer (70) über den Auslass (30) mit einer vordefinierten Flussrate entleert wird, und bei Stellung des Stellelements (90) in der zweiten Position die erste Kammer (50) entleert und die zweite Kammer (70) befüllt wird, wobei der konstante Abfluss über den Auslass mit der definierten Flussrate aufrechterhalten bleibt, sodass die Kontinuität der Förderung sichergestellt ist.

2. Infusionspumpe (10) zum kontinuierlichen Fördern eines Fluids, mit
- einem Einlass (20),
- einem Auslass (30),
- einen in einer ersten Kammer (50) verschieblich gelagerten ersten Kolben (40), und
- einem in einer zweiten Kammer (70) verschieblich gelagerten zweiten Kolben (60),
- einem die erste Kammer (50) mit der zweiten Kammer (70) verbindenden Verbindungskanal (80), wobei die erste Kammer (50) mit dem Einlass (20) und die zweite Kammer (70) mit dem Auslass (30) verbunden ist,
- einem Stellelement (90), das in einer ersten Position den Einlass (20) mit der ersten Kammer (50) und in einer zweiten Position die erste Kammer (50) mit der zweiten Kammer (70) kommunizierend verbindet, wobei in der ersten Position die Kommunikation zwischen der ersten Kammer (50) und der zweiten Kammer (70) und in der zweiten Position die Kommunikation zwischen dem Einlass (20) und der ersten Kammer (50) blockiert ist, wobei das Stellelement aus einem zwischen dem Einlass (20) und der ersten Kammer (50) angeordneten Rückschlagventil und einem zwischen der ersten Kammer (50) und der zweiten Kammer (70) im Verbindungskanal (80) angeordneten, durch einen Aktuator betätigten zweiten Ventil gebildet ist,
- einer auf den ersten Kolben (40), den zweiten Kolben (60) und das Stellelement (90) wirkenden Steuerung, sodass bei Stellung des Stellelements (90) in der ersten Position die erste Kammer (50) befüllt wird, während die zweite Kammer (70) über den Auslass (30) mit einer vordefinierten Flussrate entleert wird, und bei Stellung des Stellelements (90) in der zweiten Position die erste Kammer (50) entleert und die zweite Kammer (70) befüllt wird, wobei der konstante Abfluss über den Auslass mit der definierten Flussrate aufrechterhalten bleibt, sodass die Kontinuität der Förderung sichergestellt ist.

3. Infusionspumpe (10) nach einem der vorhergehenden Ansprüche, **gekennzeichnet durch** einen den Druck in der zweiten Kammer (70) oder im Auslass (30) erfassenden Kraftsensor (100).

4. Infusionspumpe (10) nach einem der vorhergehenden Ansprüche, **gekennzeichnet durch** einen Detektor mit einem Ultraschallsender und einem Ultraschallempfänger und/oder einen Detektor mit einer Licht abstrahlenden Lichtquelle und einem das Licht der Lichtquelle detektierenden Empfänger zum Detektieren von Luftblasen im Fluid.

5. Infusionspumpe (10) nach Anspruch 4, **dadurch gekennzeichnet, dass** der Detektor zwischen der zweiten Kammer (70) und dem Auslass (30) mit dem Fluid interagierend angeordnet ist.

6. Infusionspumpe (10) nach einem der vorhergehenden Ansprüche, **gekennzeichnet durch** ein mit dem Einlass (20) verbundenes Fluid-Reservoir.

## Claims

1. Infusion pump (10) for continuously delivering a fluid, having
- an inlet (20),
- an outlet (30),
- a first piston (40) displaceably mounted in a first chamber (50), and
- a second piston (60) displaceably mounted in a second chamber (70),
- a connecting channel (80) connecting the first chamber (50) to the second chamber (70), wherein the first chamber (50) is connected to the inlet (20) and the second chamber (70) is connected to the outlet (30),
- a control element (90) designed as a two-way or three-way stopcock, which element communicatively connects the inlet (20) to the first chamber (50) in a first position and communicatively connects the first chamber (50) to the second chamber (70) in a second position, wherein, in the first position, communication between the first chamber (50) and the second chamber (70) is blocked and, in the second position, communication between the inlet (20) and the first chamber (50) is blocked,
- a control means acting on the first piston (40), the second piston (60) and the control element (90), such that when the control element (90) is in the first position the first chamber (50) is filled, while the second chamber (70) is emptied via the outlet (30) at a predefined flow rate, and when the control element (90) is in the second position the first chamber (50) is emptied and the second chamber (70) is filled, wherein the constant outward flow via the outlet is maintained at the defined flow rate, thus ensuring continuous delivery.

2. Infusion pump (10) for continuously delivering a fluid, comprising
- an inlet (20),
- an outlet (30),
- a first piston (40) displaceably mounted in a first chamber (50), and
- a second piston (60) displaceably mounted in a second chamber (70),
- a connecting channel (80) connecting the first chamber (50) to the second chamber (70), wherein the first chamber (50) is connected to the inlet (20) and the second chamber (70) is connected to the outlet (30),
- a control element (90), which communicatively connects the inlet (20) to the first chamber (50) in a first position and communicatively connects the first chamber (50) to the second chamber (70) in a second position, wherein, in the first position, communication between the first chamber (50) and the second chamber (70) is blocked and, in the second position, communication between the inlet (20) and the first chamber (50) is blocked, wherein the control element is formed of a check valve arranged between the inlet (20) and the first chamber (50) and a second valve arranged in the connecting channel (80) between the first chamber (50) and the second chamber (70) and operated by an actuator,
- a control means acting on the first piston (40), the second piston (60) and the control element (90), such that when the control element (90) is in the first position the first chamber (50) is filled, while the second chamber (70) is emptied via the outlet (30) at a predefined flow rate, and when the control element (90) is in the second position the first chamber (50) is emptied and the second chamber (70) is filled, wherein the constant outward flow via the outlet is maintained at the defined flow rate, thus ensuring continuous delivery.

3. Infusion pump (10) according to either of the preceding claims, **characterized by** a force sensor (100) that measures the pressure in the second chamber (70) or in the outlet (30).

4. Infusion pump (10) according to any of the preceding claims, **characterized by** a detector having an ultrasonic transmitter and an ultrasonic receiver, and/or a detector having a light source that emits light and a receiver that detects the light from the light source in order to detect air bubbles in the fluid.

5. Infusion pump (10) according to claim 4, **characterized in that** the detector is arranged between the second chamber (70) and the outlet (30) so as to interact with the fluid.

6. Infusion pump (10) according to any of the preceding claims, **characterized by** a fluid reservoir connected to the inlet (20).

## Revendications

1. Pompe à perfusion (10) destinée au transport continu d'un fluide et comprenant
- une entrée (20),
- une sortie (30),
- un premier piston (40) monté de manière coulissante dans une première chambre (50), et
- un deuxième piston (60) monté de manière coulissante dans une deuxième chambre (70),
- un conduit de liaison (80) reliant la première chambre (50) à la deuxième chambre (70), la première chambre (50) étant reliée à l'entrée (20) et la deuxième chambre (70) étant reliée à la sortie (30),
- un élément de commande (90) qui est conçu comme un robinet à deux voies ou à trois voies et qui relie de manière communicante, dans une première position, l'entrée (20) à la première chambre (50) et, dans une deuxième position, la première chambre (50) à la deuxième chambre (70), la communication entre la première chambre (50) et la deuxième chambre (70) étant bloquée dans la première position et la communication entre l'entrée (20) et la première chambre (50) étant bloquée dans la deuxième position,
- une commande agissant sur le premier piston (40), le deuxième piston (60) et l'élément de commande (90) de sorte que, lorsque l'élément de commande (90) est dans la première position, la première chambre (50) se remplit, tandis que la deuxième chambre (70) se vide par la sortie (30) à un débit prédéfini et, lorsque l'élément de commande (90) est dans la deuxième position, la première chambre (50) se vide et la deuxième chambre (70) se remplit, l'évacuation constante par la sortie restant maintenue au débit défini de manière à assurer la continuité du transport.

2. Pompe à perfusion (10) destinée au transport continu d'un fluide et comprenant
- une entrée (20),
- une sortie (30),
- un premier piston (40) monté de manière coulissante dans une première chambre (50), et
- un deuxième piston (60) monté de manière coulissante dans une deuxième chambre (70),
- un conduit de liaison (80) reliant la première chambre (50) à la deuxième chambre (70), la première chambre (50) étant reliée à l'entrée (20) et la deuxième chambre (70) étant reliée à la sortie (30),
- un élément de commande (90) qui relie de manière communicante, dans une première position, l'entrée (20) à la première chambre (50) et, dans une deuxième position, la première chambre (50) à la deuxième chambre (70), la communication entre la première chambre (50) et la deuxième chambre (70) étant bloquée dans la première position et la communication entre l'entrée (20) et la première chambre (50) étant bloquée dans la deuxième position, l'élément de commande étant formé d'un clapet anti-retour disposé entre l'entrée (20) et la première chambre (50) et d'un deuxième clapet disposé dans le conduit de liaison (80) entre la première chambre (50) et la deuxième chambre (70) et actionné par un actionneur,
- une commande agissant sur le premier piston (40), le deuxième piston (60) et l'élément de commande (90) de sorte que, lorsque l'élément de commande (90) est dans la première position, la première chambre (50) se remplit, tandis que la deuxième chambre (70) se vide par la sortie (30) à un débit prédéfini et, lorsque l'élément de commande (90) est dans la deuxième position, la première chambre (50) se vide et la deuxième chambre (70) se remplit, l'évacuation constante par la sortie restant maintenue au débit défini de manière à assurer la continuité du transport.

3. Pompe à perfusion (10) selon l'une des revendications précédentes, **caractérisée par** un capteur de force (100) détectant la pression dans la deuxième chambre (70) ou dans la sortie (30).

4. Pompe à perfusion (10) selon l'une des revendications précédentes, **caractérisée par** un détecteur pourvu d'un émetteur à ultrasons et d'un récepteur à ultrasons et/ou un détecteur pourvu d'une source lumineuse émettant une lumière et d'un récepteur détectant la lumière de la source lumineuse pour détecter des bulles d'air dans le fluide.

5. Pompe à perfusion (10) selon la revendication 4, **caractérisée en ce que** le détecteur est disposé entre la deuxième chambre (70) et la sortie (30) de manière à interagir avec le fluide.

6. Pompe à perfusion (10) selon l'une des revendications précédentes, **caractérisée par** un réservoir de fluide relié à l'entrée (20).
